# EUROPÄISCHE PATENTSCHRIFT

(11) **EP 0 938 674 B1**
(45) Veröffentlichungstag und Bekanntmachung des Hinweises auf die Patenterteilung: **01.06.2005**
(21) Anmeldenummer: 97950174.9
(22) Anmeldetag: 11.11.1997
(51) Int. Cl.: G01N 33/487, G01N 21/76, G01N 33/483, C12M 1/34

(54) **MIKROELEMENTENANORDNUNG, VERFAHREN ZUM KONTAKTIEREN VON IN EINER FLÜSSIGEN UMGEBUNG BEFINDLICHEN ZELLEN UND VERFAHREN ZUM HERSTELLEN EINER MIKROELEMENTENANORDNUNG**
ARRAY OF MICROELEMENTS, METHOD OF CONTACTING CELLS IN A LIQUID ENVIRONMENT AND METHOD FOR THE PRODUCTION OF AN ARRAY OF MICROELEMENTS
SYSTEME DE MICROELEMENTS, PROCEDE DE MISE EN CONTACT DE CELLULES SITUEES DANS UN ENVIRONNEMENT LIQUIDE ET PROCEDE PERMETTANT DE REALISER UN SYSTEME DE MICROELEMENTS

(30) Priorität: 16.11.1996 DE 19647525; 24.03.1997 DE 19712309
(43) Veröffentlichungstag der Anmeldung: 01.09.1999
(73) Patentinhaber: NMI Naturwissenschaftliches und Medizinisches Institut an der Universität Tübingen in Reutlingen Stiftung Bürgerlichen Rechts, 72770 Reutlingen (DE)
(72) Erfinder: NISCH, Wilfried, D-72072 Tübingen (DE); STETT, Alfred, D-72768 Reutlingen (DE); EGERT, Ulrich, D-72770 Reutlingen (DE); STELZLE, Martin, D-72770 Reutlingen (DE)
(74) Vertreter: Gahlert, Stefan, Dr.-Ing.
(86) Internationale Anmeldenummer: PCT/EP1997/006285
(87) Internationale Veröffentlichungsnummer: WO 1998/022819

(56) Entgegenhaltungen:
- EP-A- 0 094 193
- EP-A- 0 689 051
- WO-A-85/02201
- WO-A-96/32467
- US-A- 5 173 158

## Beschreibung

Die Erfindung betrifft eine Mikroelementenanordnung mit einer Vielzahl von auf einem Substrat angeordneten Mikroelementen zum Kontaktieren von in einer flüssigen, vorzugsweise biologischen Umgebung befindlichen Zellen.

Die Erfindung betrifft ferner ein Verfahren zum Kontaktieren von in einer flüssigen, vorzugsweise biologischen Umgebung oberhalb eines Substrats befindlichen Zellen, bei dem ein Kontakt zwischen den Zellen und Mikroelementen hergestellt wird.

Die Erfindung betrifft schließlich ein Verfahren zum Herstellen einer Mikroelementenanordnung mit einer Vielzahl von Mikroelektroden, bei dem die Mikroelemente auf einem Substrat angeordnet werden.

Aus der EP-A-0 689 051 ist eine Mikroelektrodenanordnung bekannt, die zur Aufnahme von in einer flüssigen Umgebung befindlichen Zellen und zur Kontaktierung derselben geeignet ist, um Zellpotentiale messen zu können. Zur Zuführung der in einer flüssigen Umgebung befindlichen Zellen an die Mikroelektroden werden Pipetten oder dgl. verwendet.

Mit einer derartigen Anordnung und einem derartigen Verfahren ist es nicht möglich, gezielt einzelne Zellen in Kontakt mit den Kontaktierungselektroden zu bringen.

Aus der WO-A-85 02 201 ist ein System und ein Verfahren zur Zellenselektion bekannt. Durch die Verwendung eines Trägers, der eine exakte Anordnung von Vertiefungen, die eine individuelle Zelle aufnehmen können, besitzt, können Einzelzellen an genau lokalisierbaren Orten abgelegt werden.

Aus der WO-A-96 32 467 ist schließlich eine Vorrichtung zur Untersuchung von elektro-physiologischen und biochemischen Aktivitäten einer Gewebekultur bekannt, bei der jedoch keine gezielte Führung einzelner Zellen zu den Meßelektroden möglich ist.

Mikroelektrodenanordnungen zum Untersuchen von biologischen Zellen dienen z.B. zum Stimulieren der Zellen oder zum Ableiten von Potentialen. Die Untersuchungen können dabei in einer biologischen Umgebung durchgeführt werden oder auch in einer artiziellen Umgebung. Diese kann z.B. eine Suspension mit künstlichen Vesikeln aus Lipiden sein, wobei in die Vesikelhülle Poren als Modellsystem für biologische Zellen eingebaut sind. Die Anordnungen umfassen hierzu auf einem Substrat eine Vielzahl von Mikroelektroden, deren Abmessungen etwa in der Größenordnung der Zellen liegen, also im Bereich von einigen µm bis einige 10 µm.

Aus der prioritätsälteren, jedoch nicht vorveröffentlichten deutschen Patentanmeldung P 195 29 371 ist eine derartige Mikroelektrodenanordnung bekannt.

Zur Messung der Bio-/Chemolumineszenz, z.B. als Reaktion auf chemische Reize (Noxen, Pharmaka) sowie zur Messung von Änderungen der Lichtabsorption durch einen solchen Reiz bei Verwendung einer Lichtquelle oberhalb der Zellen können lichtempfindliche Mikroelemente eingesetzt werden, z.B. Mikrophotodioden, die für bestimmte Spektralbereiche empfindlich sind.

Mikroelektroden, Mikrophotodioden und dgl. werden im Rahmen der vorliegenden Erfindung gesamthaft als "Mikroelemente" bezeichnet.

Bei Mikroelektrodenanordnungen herkömmlicher Art sowie den damit ausgeführten Verfahren stellen sich unter anderem die folgenden Probleme:

Wenn die Mikroelektrodenanordnung in Kontakt mit einer Suspension, d.h. einer flüssigen, z.b. einer biologischen Umgebung gebracht wird, in der sich Zellen befinden, ist man mehr oder weniger auf den Zufall angewiesen, daß die eine oder die andere Zelle sich auf einer bestimmten Elektrode niederläßt. In der Praxis lassen sich die Zellen im allgemeinen nur unter teilweiser Überdeckung auf einer Elektrode nieder, so daß die Stimulation der Zelle bzw. die Ableitung eines Zellpotentials auf diese Teilfläche beschränkt ist. Bei einer Stimulation der Zelle geht dabei bspw. ein Teil der Stimulationsenergie in der als Elektrolyt wirkenden Suspension verloren.

Darüber hinaus sitzen die Zellen nur lose auf den Elektroden auf. Dies kann zu Problemen hinsichtlich des Abdichtwiderstandes zur Referenzelektrode führen. Darüber hinaus ist der Kontakt sehr empfindlich und wird bereits bei äußerst geringen mechanischen Beeinflussungen wieder gestört, weil sich die Zelle vom Kontakt löst.

Der Erfindung liegt daher die Aufgabe zugrunde, eine Mikroelementenanordnung, ein Verfahren zum Kontaktieren sowie ein Verfahren zum Herstellen einer derartigen Mikroelementenanordnung dahingehend weiterzubilden, daß die vorstehend genannten Probleme vermieden werden. Insbesondere soll es möglich werden, aus der flüssigen Umgebung gezielt einzelne Zellen in Kontakt mit den Mikroelementen zu bringen und dort einen guten Kontakt herzustellen.

Bei der eingangs genannten Mikroelementenanordnung wird diese Aufgabe erfindungsgemäß durch Mittel zum Führen und/oder Vereinzeln und/oder mechanischen Anziehen der Zellen an die Mikroelementen gelöst.

Bei dem eingangs zunächst genannten Verfahren zum Kontaktieren wird die Aufgabe erfindungsgemäß dadurch gelöst, daß eine Führungs- und/oder Anziehungskraft zwischen den Zellen und den Mikroelementen bzw. dem Substrat erzeugt wird.

Schließlich wird bei dem eingangs als zweites genannten Verfahren zum Herstellen einer Mikroelementenanordnung die Aufgabe erfindungsgemäß dadurch gelöst, daß das Substrat mindestens aus einer Grundplatte und einer darüberliegenden Deckplatte hergestellt wird.

Die zugrundeliegende Aufgabe wird vollkommen gelöst.

Dadurch, daß die Zellen mechanisch an die Mikroelemente angezogen werden, ergeben sich nämlich zwei Vorteile:

Solange die Zellen sich noch frei in der flüssigen Umgebung befinden, bewirkt die Anziehungskraft, daß sich die Zellen gezielt auf die Elemente zu bewegen. Man ist daher nicht mehr auf den Zufall angewiesen, daß sich die eine oder die andere Zelle auf einem vorbestimmten Mikroelement niederlassen würde.

Wie bereits erwähnt wurde, bezieht sich die vorliegende Anmeldung auf Mikroelemente, d.h. vorzugsweise Mikroelektroden oder Mikrophotodioden, ohne auf diese Anwendung eingeschränkt zu sein. Dies betrifft insbesondere auch die weiter unten geschilderten Ausführungsbeispiele, bei denen von Mikroelektroden die Rede ist, wobei jedoch die Ausführungen zumeist auch für Mikrophotodioden und ähnliche Mikroelemente gelten.

Zum anderen bewirkt eine fortwährende Anziehungskraft im Falle einer Mikroelektrode, daß die Zeilen mit einer gewissen Kontaktkraft gegen die Mikroelektroden gedrückt werden und sich dadurch ein besonders guter Abdichtwiderstand zur Referenzelektrode ergibt, und die Anhaftung darüber hinaus auch mechanisch stabil ist. Der elektrische Widerstand und damit das meßbare Signal (Aktionspotential) wird wesentlich verbessert .

Aus einem anderen Fachgebiet, der sogenannten Patch-Clamp-Technik ist es zwar bekannt, Zellen an eine Pipette mit Unterdruck anzusaugen (vgl. US-Z "Nature", Vol. 260, S. 799-801, 1976), bei der Patch-Clamp-Technik muß jedoch die Pipette gezielt an eine einzelne Zelle herangeführt werden. Bei der Patch-Clamp-Technik werden die zu kontaktierenden Zellen nicht bewegt, da sie in der Regel am Substrat anhaften. Das Kontaktieren von Zellen mit Patch-Clamp-Pipetten wird wesentlich erleichtert, wenn die Zellen durch Adhäsion immobilisiert sind. Translokalisation adhärierender Zellen führt fast immer zu letalen Zellschädigungen. Der Hauptnachteil der Patch-Clamp-Technik liegt in der Beschränkung der Anzahl gleichzeitig kontaktierbarer Zellen, da aus Platzgründen nicht beliebig viele Pipetten in die Kulturkammer eingeführt werden können. Die Erfindung hat demgegenüber den Vorteil, daß eine Vielzahl von Zellen gleichzeitig kontaktiert werden kann, ohne daß die erwähnten Platzprobleme auftreten.

Die Verwendung einer Grundplatte sowie einer davon getrennten Deckplatte hat im Rahmen der vorliegenden Erfindung insbesondere Vorteile bei wiederholter Verwendung der erfindungsgemäßen Anordnung. So können z.B. die Grundplatte und die Deckplatte jeweils einzeln oder zusammen mehrfach wiederverwendet werden. Für die beiden Platten können darüber hinaus unterschiedliche Herstellungsverfahren und Materialien eingesetzt werden.

Schließlich ergibt sich ein Vorteil bei der Ausformung der Elektrodengeometrie durch die Geometrie (die Öffnungen) der Deckplatte sowie eine dadurch vereinfachte Herstellung.

Bei einer bevorzugten Ausgestaltung der erfindungsgemäßen Anordnung üben die Mittel eine Unterdruck-Kraft auf die Zellen aus.

Diese Maßnahme hat den Vorteil, daß durch rein mechanische Mittel, nämlich durch Erzeugen eines Unterdrucks bzw. Vakuums, die erforderliche Anziehungskraft erzeugt werden kann.

Bei einem anderen Ausführungsbeispiel der Erfindung üben die Mittel eine hydrodynamische Kraft auf die Zellen aus.

Diese Maßnahme hat den Vorteil, daß durch das Erzeugen einer Strömung in der flüssigen biologischen Umgebung die gewünschte Kraft ebenfalls auf einfache Weise erzeugt werden kann.

Bevorzugt ist ferner, wenn die Mittel Kanäle umfassen, die in einer Kontaktoberfläche der Mikroelemente ausmünden.

Diese Maßnahme hat den Vorteil, daß die Zellen an die Mikroelemente herangeführt und dort festgehalten werden können, wobei gleichzeitig praktisch eine Zentrierung der Zellen auf den Mikroelementen möglich ist.

Bei diesem Ausführungsbeispiel ist ferner bevorzugt, wenn die Kanäle mit einer Unterdruckquelle verbindbar sind.

Dann kann in der bereits erwähnten Weise die Zelle mittels einer Unterdruck-Kraft an die Kontaktoberfläche herangeführt und dort festgehalten werden.

Bei dem alternativen Ausführungsbeispiel sind die Kanäle mit einer Pumpeinrichtung für die flüssige Umgebung verbunden. Die Pumpeinrichtung ist vorzugsweise als Elektroosmose-Einrichtung ausgebildet.

Diese Maßnahme hat den Vorteil, daß der erforderliche hydrodynamische Fluß durch einfache Elektroosmose erzeugt werden kann. Bei der Elektroosmose kann bei gleichem Kanalquerschnitt ein im Vergleich zu normalen Unterdruckeinrichtungen erheblich größerer hydrodynamischer Fluß der Suspension erzeugt werden. Man kann daher auf diese Weise Zellen aus der weiteren Umgebung der Kontaktoberfläche mit hoher Wirksamkeit anziehen. Elektroosmotisches Pumpen wird umso vorteilhafter gegenüber pneumatischen Pumpen, je kleiner der Kanalquerschnitt ist.

Bei einer bevorzugten Weiterbildung dieses Ausführungsbeispiels umfaßt die Pumpeinrichtung als Elektroosmose-Einrichtung zwei Elektroden, die an entgegengesetzten Enden der Kanäle wirksam sind, wobei zwischen die Elektroden eine Spannung geschaltet ist.

Diese Maßnahme hat den Vorteil, daß die gewünschte Elektroosmose-Einrichtung mit extrem einfachen Bauelementen realisiert wird.

Ferner ist bevorzugt, wenn die Mittel eine elektrostatische Kraft auf die Zellen ausüben.

Auch diese Maßnahme hat den Vorteil, daß mit einfachen technischen Mitteln die gewünschte Kraft auf die Zellen ausgeübt wird, um diese zu führen, zu vereinzeln oder anzuziehen.

Bei bevorzugten Ausführungsbeispielen der Erfindung, die auch isoliert und in anderem Zusammenhang eingesetzt werden können, sind Mittel zum Führen und/oder Vereinzeln der Zellen vor dem mechanischen Anziehen an die Mikroelemente vorgesehen.

Diese Maßnahme hat den Vorteil, daß einzelne Zellen gezielt an die Mikroelemente herangeführt werden können, so daß sich beim Kontaktieren der Zellen definierte Verhältnisse ergeben.

Bevorzugt umfassen die Mittel trichterartige Mikroküvetten im Substrat, wobei sich die Mikroelemente am Boden der Mikroküvetten befinden.

Diese Maßnahme hat den Vorteil, daß die Zellen von den trichterartigen Mikroküvetten eingefangen und gezielt auf die vorzugsweise ringförmigen Elektroden aufgesetzt werden. Dies geschieht unabhängig davon, ob die Zellen angezogen werden oder ob sie infolge der Schwerkraft passiv absinken. Die Zellen werden dadurch auch mechanisch auf den Mikroelektroden zentriert.

Bevorzugt ist, wenn Oberflächenbereiche zwischen den Mikroküvetten mit einem zellabweisenden Substrat beschichtet sind.

Diese Maßnahme hat den Vorteil, daß ein passives Absinken der Zellen infolge Schwerkraft auf die Zwischenräume zwischen den Küvetten verhindert werden kann, indem die Oberfläche im Bereich dieser Zwischenräume mit einem repulsiven, d.h. einem zellabweisenden Substrat beschichtet wird. Die Zellen senken sich dann bevorzugt in die Trichter, d.h. die Küvetten, ab und haften dann auf den Küvettenböden. Dies gilt insbesondere dann, wenn diese zusätzlich mit einem attraktiven Substrat beschichtet sind.

Erfindungsgemäß besteht das Substrat vorzugsweise mindestens aus einer Grurdplatte und einer darüberliegenden Deckplatte.

Diese Maßnahme hat den Vorteil, daß die Mittel zum mechanischen Anziehen, der Zellen als Einzelelemente, bspw. als Kanalsystem, als Elektroden, Mikroküvetten usw. definiert auf der Grundplatte und/oder der Deckplatte vorgesehen werden können.

Bevorzugt bestehen die Grundplatte und/oder die Deckplatte aus Quarz, Glas, Silizium oder Kunststoff, insbesondere aus Polystyrol, PMMA oder Polyimid.

Weiterhin ist bevorzugt, wenn die Grundplatte und/oder die Deckplatte aus einem für Licht durchlässigen Material bestehen, wobei die Wellenlänge des Lichtes in einem für Mikroskopietechniken zugänglichen Bereich des Spektrums liegt.

Diese Maßnahme hat den Vorteil, daß eine optische Beobachtung der Experimente über ein Mikroskop oder dgl. möglich ist.

Bei Ausführungsbeispielen der Erfindung mit aufeinandergeschichteten Platten ist bevorzugt, wenn die mit den Mikroelementen versehene Grundplatte seitlich als Steckerleiste herausgeführt ist.

Diese Maßnahme hat den Vorteil, daß ein einfacher elektrischer Zugriff auf die Mikroelemente von außen her möglich ist und daß die Anordnung insgesamt problemlos in übliche standardisierte elektrische Meßaufbauten integriert werden kann.

Besonders bevorzugt ist in diesem Zusammenhang ferner, wenn die Grundplatte mindestens aus einer unteren Signalverarbeitungs-Platte und einer darüberliegenden Elementen-Platte besteht.

Diese Maßnahme hat den Vorteil, daß die sehr schwachen von den Zellen abgeleiteten Meßsignale auf kurzem Wege bereits verarbeitet werden können, so daß ein hohes Signal/Rausch-Verhältnis erreicht werden kann.

Auch in diesem Falle ist es analog vorteilhaft, wenn die Signalverarbeitungs-Platte seitlich als Steckerleiste herausgeführt ist.

Eine gute Wirkung wird ferner dann erzielt, wenn die Mikroelektroden Ableitelektroden sowie Reizelektroden und/oder Referenzelektroden umfassen.

Eine solche Mehrfachelektrodenanordnung hat den Vorteil, daß sehr unterschiedliche Experimente unter reproduzierbaren Bedingungen durchgeführt werden können.

Bevorzugt sind die mehreren Elektroden dabei konzentrisch zueinander angeordnet.

Sofern eine Referenzelektrode vorgesehen ist, wird diese vorzugsweise im Abstand oberhalb der am Boden der Mikroküvette angeordneten Ableitelektrode angeordnet .

Bei einer bevorzugten Weiterbildung der erfindungsgemäßen Anordnung ist die mit der Umgebung in Kontakt befindliche Oberfläche der Mikroelektrode größer als die mit der Zelle in Kontakt befindliche Oberfläche.

Diese Maßnahme hat den Vorteil, daß die sogenannte Helmholtz-Kapazität vermindert wird. Für die Helmholtz-Kapazität ist nämlich die Oberfläche zwischen Elektrode und Elektrolyt, d.h. der Umgebung, maßgeblich, nicht hingegen die Oberfläche, die mit der Zelle in Kontakt steht.

Bei einer bevorzugten Weiterbildung dieses Ausführungsbeispiels ist die Mikroelektrode als Kammer in einem Substrat ausgebildet, wobei die Kammer über eine Öffnung in den das Substrat umgebenden Außenraum mündet.

Diese Maßnahme hat den Vorteil, daß in Gestalt eines Kanals oder eines verschlossenen Hohlraums Elektroden großer Oberfläche versenkt werden können, indem die entsprechende Oberfläche des Kanals oder Hohlraums z.B. vergoldet wird. Der Abdichtwiderstand gegen die Referenzelektrode wird dabei durch die Abdichtung der zellseitigen Kanal- oder Hohlraumöffnung bestimmt, die kleingehalten werden kann. Durch diese Anordnung können kleinere Impedanzen und damit bessere Ableiteigenschaften realisiert werden. Statt eines Hohlraumes oder eines Kanals mit vergoldeter Oberfläche kann z.B. auch ein Schwamm aus einem Edelmetall, bspw. ein Platinschwamm, verwendet werden. Diese Anordnung ist auch außerhalb des Rahmens der vorliegenden Erfindung einsetzbar.

Bei Ausführungsbeispielen des erfindungsgemäßen Verfahrens zum elektrischen Kontaktieren wird die Kraft, wie bereits erwähnt, vorzugsweise als Unterdruck-Kraft oder als hydrodyamische Kraft, letztere vorzugsweise mittels Elektroosmose, oder als elektrostatische Kraft ausgeübt.

Auf diese Weise kann eine Kontaktkraft zwischen Zellen und Mikroelektrode und/oder eine Kraft für eine gerichtete Bewegung der Zellen auf die Mikroelektroden zu ausgeübt werden.

Bei bevorzugten Varianten des Verfahrens werden die Zellen über die Mikroelektroden stimuliert oder es werden über die Mikroelektroden Potentiale von den Zellen abgeleitet. Alternativ können über die als Mikrophotodioden ausgebildeten Mikroelemente die Lumineszenz der Zellen und/oder deren Lichtabsorption gemessen werden, wie weiter oben bereits erläutert .

Bei dem erfindungsgemäßen Verfahren zum Herstellen einer Mikroelektrodenanordnung wird vorzugsweise in der Grundplatte ein Kanalsystem ausgebildet, in der Deckplatte werden Mikroküvetten ausgeformt und die Grundplatte wird mit der Deckplatte derart zusammengefügt, daß Öffnungen am Boden der Mikroküvetten in Kontaktoberflächen der Mikroelemente angeordnet sind und mit dem Kanalsystem kommunizieren.

Diese Maßnahmen haben den Vorteil, daß mit an sich bekannten und beherrschbaren Mikrostrukturtechniken die erforderlichen Elemente für die Mittel zum mechanischen Anziehen der Zellen in der Mikroelementenanordnung hergestellt werden können.

Bei einem bevorzugten Ausführungsbeispiel dieses Verfahrens sind folgende Schritte vorgesehen:
a) Versehen von Grundplatte und/oder Deckplatte auf ihren einander zuweisenden Oberflächen mit einer Schicht von Molekülen mit reaktiver Endgruppe;
b) Zusammenfügen von Grundplatte und Deckplatte; und
c) Aktivieren einer kovalenten Bindung der Schichten mittels eines äußeren Stimulus.

Diese Maßnahme hat den Vorteil, daß die Montage von Grundplatte und Deckplatte aufeinander in präziser Weise möglich ist, wobei auf mechanische Verbindungselemente und dgl. verzichtet werden kann.

Besonders bevorzugt ist dabei, wenn nach Schritt b) die Grundplatte und die Deckplatte relativ zueinander justiert werden.

Diese Maßnahmen machen sich mit Vorteil zunutze, daß vor dem Aktivieren der kovalenten Bindung noch ein Verschieben von Grundplatte und Deckplatte relativ zueinander möglich ist. Man kann daher die beiden Teile relativ zueinander in einem Maskaligner oder einer ähnlichen Apparatur ausrichten.

Erst anschließend wird der äußere Stimulus ausgeübt, bevorzugt als Temperatur, Licht oder als elektrisches Feld.

Bei einer weiteren Variante des erfindungsgemäßen Herstellverfahrens werden die Grundplatte und die Deckplatte durch anodisches oder metallisches Bonden miteinander verbunden.

Weitere Vorteile ergeben sich aus der Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der beigefügten Zeichnung dargestellt und werden in der nachfolgenden Beschreibung näher erläutert. Es zeigen:
- Fig. 1A und 1B: eine äußerst schematisierte Querschnittsdarstellung eines Ausführungsbeispiels einer erfindungsgemäßen Mikroelektrodenanordnung in zwei unterschiedlichen Betriebsphasen;
- Fig. 2: eine Darstellung, ähnlich Fig. 1A und 1B, jedoch für eine Mikroelektrodenanordnung nach dem Stand der Technik;
- Fig. 3: eine weitere Darstellung, ähnlich Fig. 1A und 1B, jedoch für ein anderes Ausführungsbeispiel der Erfindung;
- Fig. 4: eine Draufsicht auf ein weiteres Ausführungsbeispiel einer erfindungsgemäßen Mikroelektrodenanordnung;
- Fig. 5 und 6: in vergrößertem Maßstab zwei Darstellungen von Schnitten durch Mikroküvetten, wie sie bei der Anordnung gemäß Fig. 5 verwendet werden können; und
- Fig. 7: in einer Querschnittsdarstellung eine weitere Ausführungsform einer erfindungsgemäßen Mikroelektrode.

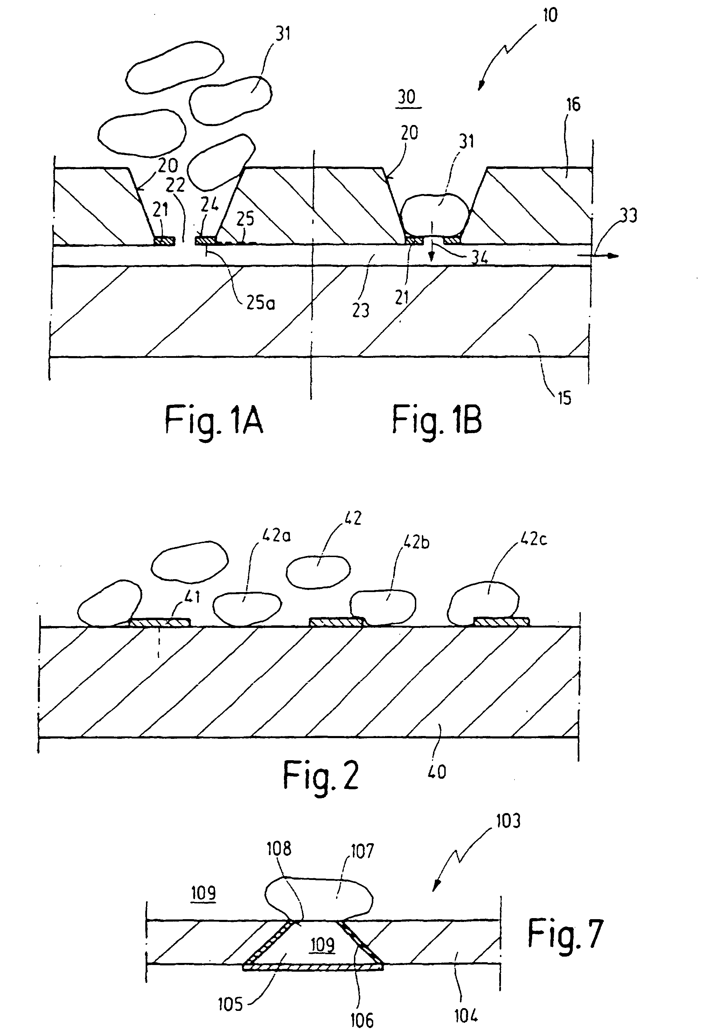

In Fig. 1 bezeichnet 10 als Ganzes eine Mikroelementenanordnung. Die Anordnung besteht im wesentlichen aus einem zweischichtigen Substrat mit einer Grundplatte 15 und einer Deckplatte 16. Wie bereits erwähnt wurde, sind die nachfolgend geschilderten Mikroelektroden nur als Beispiele für Mikroelemente unterschiedlicher Art zu verstehen. Die Erfindung ist also nicht auf den Anwendungsbereich der Mikroelektroden beschränkt.

In der Deckplatte 16 sind trichterartige Mikroküvetten 20 angebracht. Die Mikroküvetten 20 laufen an ihrer Unterseite in Ringelektroden 21 aus. Die Ringelektroden 21 münden mit ihrer zentralen Öffnung in einen gemeinsamen Kanal 23 oder können einzeln nach außen geführt werden.

Der Kanal 23 wird vorzugsweise mit mikrotechnischen Verfahren so geformt, daß ein Graben in die Grundplatte geätzt wird. Der Kanal 23 bildet sich dann durch Auflegen der Deckplatte.

Die Oberseite der Ringelektroden 21 dienen als Kontakt-Oberflächen 24. Die Ringelektroden 21 können z.B. mittels Leiterbahnen 25 in der Trennebene zwischen Grundplatte 15 und Deckplatte 16 anschließbar sein, aber auch andere Leiterführungen sind möglich, wie mit 25a angedeutet.

Oberhalb der Anordnung 10 befindet sich eine mit 30 angedeutet flüssige biologische Umgebung oder Suspension oder Pufferlösung, in der sich biologische Zellen 31 befinden. Auch hier ist der Fall einer biologischen Umgebung als Elektrolyt nur beispielhaft zu verstehen. Möglich ist im Rahmen der vorliegenden Erfindung auch die Verwendung einer Suspension mit künstlichen Vesikeln aus Lipiden, wobei in die Vesikelhülle Poren als Modellsystem für biologische Zellen eingebaut sind. Die Suspension stellt dann keine flüssige biologische sondern vielmehr eine flüssige artifizielle Umgebung dar.

Wie aus Fig. 1A erkennbar, befinden sich die Zellen 31 ungeordnet in der Umgebung 30.

Wenn nun, wie mit einem Pfeil 33 angedeutet, ein Unterdruck an den gemeinsamen Kanal 23 angelegt wird, werden Zellen 31 in Richtung auf die Ringelektroden 21 zu angesaugt.

Aus Fig. 1B ist erkennbar, daß eine Zelle 31 infolge des wirksamen Unterdrucks auf der Ringelektrode 21 aufsitzt und dort festgehalten wird, wie mit einem Pfeil 34 angedeutet.

Die Mikroküvetten 20 bewirken dabei, daß die Zellen 31 auf den Ringelektroden 21 bzw. den Kontakt-Oberflächen 24 zentriert werden. Auf diese Weise ist die Kontaktfläche zwischen den Zellen und den Mikroelektroden besonders groß.

Im Gegensatz dazu ist in Fig. 2 eine herkömmliche Anordnung dargestellt. Auf einem Substrat 40 sitzen vereinzelte Elektroden 41. Mehr oder weniger zufällig setzen sich nun Zellen 42 auf den Elektroden 41 ab. Eine Zelle 42a in Fig. 2 sitzt bspw. nur auf dem Substrat 40 und hat keinerlei Kontakt mit einer Elektrode 41. Zellen 42b und 42c sitzen bspw. unter teilweiser Überlappung auf Elektroden 41, wobei das Überlappungsverhältnis ebenfalls zufällig ist.

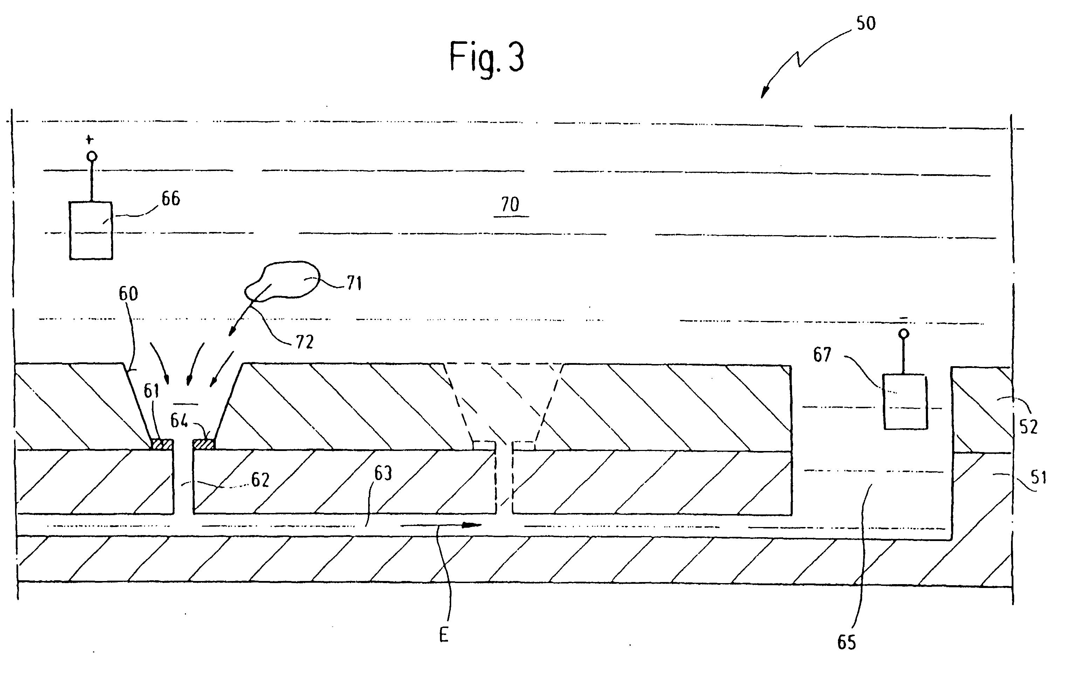

In Fig. 3 ist ein weiteres Ausführungsbeispiel der Erfindung dargestellt.

Eine Mikroelektrodenanordnung 50 umfaßt wiederum eine Grundplatte 51 und eine Deckplatte 52. In der Deckplatte 52 sind wiederum Mikroküvetten 60 ausgeformt, an deren Boden sich Ringelektroden 61 mit Kontaktoberflächen 64 befinden.

Die Grundplatte 51 umfaßt ein Kanalsystem mit Stichkanälen 62, die zentral in den Ringelektroden 61 ausmünden. Die Stichkanäle 62 sind wiederum an einen gemeinsamen Kanal 63 angeschlossen. Der gemeinsame Kanal 63 kann auch hier (vgl. Fig. 1) als Graben ohne Stichkanäle ausgebildet sein.

Insoweit entspricht das Ausführungsbeispiel gemäß Fig. 3 dem gemäß Fig. 1A und 1B.

In Abweichung dazu ist der gemeinsame Kanal 63 an ein Reservoir 65 angeschlossen. Oberhalb der Deckplatte 52 befindet sich eine erste Elektrode 66. Im Reservoir 65 befindet sich eine zweite Elektrode 67. Zwischen die Elektroden 66, 67 ist eine Spannung geschaltet, die mit "+" und "-" angedeutet ist.

Wenn die Spannung zwischen die Elektroden 66 und 67 gelege wird, entsteht ein elektrisches Feld E tangential zu den Wänden des Kanals 63, wie mit "E" in Fig. 3 eingezeichnet. Dies wiederum führt in dem Elektrolyt-gefüllten Kanal 63 zu einem Elektrolyttransport und damit zu einer hydrodynamischen Strömung. Die oberhalb der Deckplatte 52 befindliche Suspension, die in Fig. 3 mit 70 angedeutet ist, strömt dann auf die Mikroküvetten 60 zu. Auf diese Weise wird auf Zellen 71 in der Suspension 70 eine Kraft ausgeübt, wie mit einem Pfeil 72 angedeutet.

Die Zellen 71 setzen sich dann zentriert auf den Ringelektroden 61 ab, wie dies bereits in Fig. 1B für das dort beschriebene Ausführungsbeispiel dargestellt wurde.

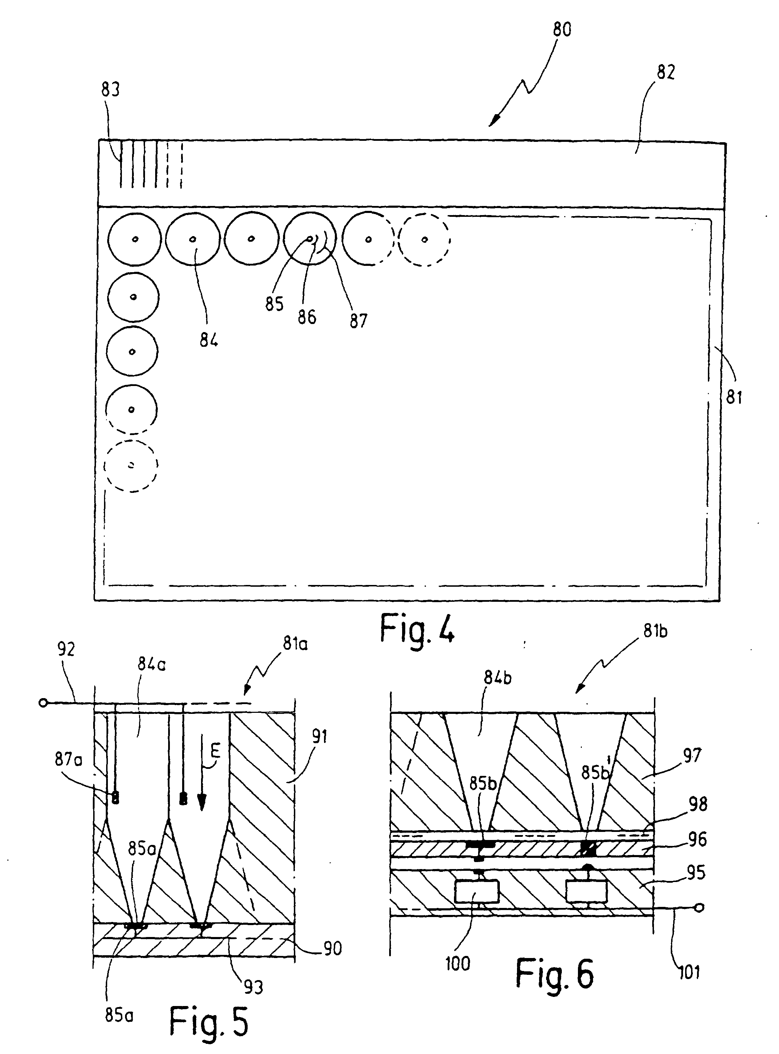

Fig. 4 zeigt in der Draufsicht ein weiteres Ausführungsbeispiel einer Mikroelektrodenanordnung 80. Diese besteht aus einer Plattenanordnung 81, aus der seitlich eine Steckerleiste 82 mit Kontaktzungen 83 vorsteht.

In der Plattenanordnung 81 sind von oben Küvetten eingelassen, bspw. 8 x 12 = 96 Küvetten, wobei diese Anzahl auch wesentlich größer oder kleiner sein kann.

In einer der Küvetten 84 sind eine Ableitelektrode 85, eine Reizelektrode 86 sowie eine Referenzelektrode 87 angedeutet. Die Elektroden 85, 86, 87 sind vorzugsweise konzentrisch zueinander angeordnet.

Die Plattenanordnung 80 ist im Aufbau mehrschichtig, wie bereits weiter oben erläutert wurde. Die Fig. 5 und 6 zeigen im Schnitt zwei Varianten des Schichtenaufbaus.

Bei der Variante gemäß Fig. 5 sind eine Grundplatte 90 sowie eine Deckplatte 91 vorgesehen. In der Grundplatte 90 befinden sich mindestens die Ableitelektroden 85, während in der Küvette 84a eine Referenzelektrode 87a im Abstand oberhalb der Ableitelektrode 85a angeordnet ist.

Die Ableitelektroden 85a sind mit einer Leitung 93 verbunden. die Referenzelektroden 87a mit einer Leitung 92. Es versteht sich, daß die Leitungsführung hier und auch bei den anderen Figuren nur äußerst schematisch zu verstehen ist. Die Leitungen können als Einfachleitungen. Mehrfachleitungen oder als im Multiplexbetrieb verwendete Leitungen ausgebildet sein.

In Fig. 5 ist ferner mit einem Pfeil ein elektrisches Feld E angedeutet, das ebenfalls verwendet werden kann, um eine elektrostatische Kraft auf Zellen auszuüben, die sich dann an den schrägen Flächen der Küvette 84a nach unten führen lassen und schließlich auf die Ableitelektroden 85a sinken. Im allgemeinen wird aber die Wirkung der Schwerkraft ausreichen.

Bei der Variante gemäß Fig. 6 wird eine mindestens dreischichtige Anordnung verwendet. Auf einer Signalverarbeitungs-Platte 95 befindet sich eine Elektroden-Platte 96. Oberhalb dieser ist - ggf. über eine Dichtung 98 - eine Deckplatte 97 angeordnet.

In der Signalverarbeitungs-Platte 95 befinden sich Verstärker 100, ggf. inklusive Impedanz-Wandlern, Filtern, Signalanalysatoren oder Anpassung-Bauelementen, wobei die Verstärker 100 über Leitungen 101 mit der Umgebung verbunden sind.

In der Elektroden-Platte 96 befinden sich mindestens Ableitelektroden 85b, 85b', die - wie dargestellt - flach oder stabförmig oder dgl. ausgebildet sein können.

In der Deckplatte 97 befinden sich schließlich die bereits mehrfach erwähnten Küvetten 84b. Selbstverständlich können auch hier Referenzelektroden an verschiedenen Orten vorgesehen sein.

Bei den Ausführungsbeispielen besteht die Elektrodenanordnung 10 bzw. 50 bzw 80, wie erwähnt, jeweils aus einer Grundplatte 15; 51; 90; 95, 96 sowie einer Deckplatte 16; 52; 91; 97.

Die Platten können mit geeigneten Strukturen (Leiterbahnen, Elektroden usw.) versehen und danach zusammengebondet werden. Dies kann entweder durch konventionelles metallisches Bonden unter Ausnutzung der Leiterbahnen (vgl. 25 in Fig. 1A) oder mit Hilfe von dünnen organischen Schichten geschehen.

Im letztgenannten Fall werden z.B. photochemisch oder thermisch aktivierbare Gruppen verwendet (Beispiele in: US-Z "Int. J. Peptide Protein Res., Vol. 47, S. 419-426, 1996), die eine lichtinduzierte Kopplung beider Platten ermöglichen. Zum Herstellen der Anordnungen 10; 50 werden die Platten auf ihren einander zuweisenden Oberflächen mit jeweils einer ultradünnen Schicht, die kovalent an die jeweilige Oberfläche gekoppelt ist, von bspw. 10 nm Dicke aus Molekülen mit reaktiven Endgruppen versehen. Diese Schichten gestatten eine kovalente Verbindung zwischen Grundplatte und Deckplatte durch einen äußeren Stimulus, z.B. Temperatur, Licht oder ein elektrisches Feld. Vor dem Ausüben des Stimulus können die Platten noch relativ zueinander verschoben und damit ausgerichtet werden, z.B. in einem Maskaligner, wie er auch in der Photolithographie verwendet wird. Es sind aber auch andere Verfahren denkbar.

Die Platten können aus einem Polymer mit Hilfe einer Stempeltechnik geformt sein. Sie können auch durch übliche Mikrostrukturtechniken hergestellt werden.

Die am Boden der Mikroküvetten 20 bzw. 60 vorgesehenen Ringelektroden 21 bzw. 61 bestehen bevorzugt aus. TiN, Iridium, Iridiumoxid, Platin, Platinmohr oder Gold. Sie können mit einer dünnen Schicht chemisch funktionalisiert sein, so daß vorzugsweise eine spezifische Wechselwirkung mit den anzuhaftenden Zellen induziert wird.

Besonders bevorzugt ist, wenn die Mikroelektroden als ionensensitive Elektroden ausgeführt sind.

Wenn die Elektroden mit einer speziellen Oberflächenbeschichtung versehen werden, führt dies in spezifischer Weise zu einer elektrisch abdichtenden Wechselwirkung mit der Zellmembran. Hier sind bspw., aber nicht beschränkt auf solche, lipidähnliche Moleküle, Zelladhäsionsproteine oder -peptide, Glycoproteine oder -peptide und hydrophobe Beschichtungen zu nennen.

In Fig. 7 ist schließlich noch ein weiteres Ausführungsbeispiel einer erfindungsgemäßen Mikroelektrodenanordnung 103 dargestellt. In einem Substrat 104 befindet sich eine Kammer 105 von z.B. kegelstumpfförmiger Gestalt. Die Kammer 105 ist an ihren Wänden mit einer leitenden Beschichtung 106 versehen, insbesondere vergoldet. Die Kammer 105 kann, wie dargestellt, nach unten abgeschlossen und mit einem Anschluß versehen sein. Sie kann aber alternativ auch an die Stelle eines der Kanäle treten, wie sie weiter oben geschildert wurden (bspw. Kanal 62 in Fig. 3) .

Eine Zelle 107 liegt oben auf der Öffnung 108 der Kammer 105 auf. Da die Kammer 105 vor dem Aufliegen der Zelle 107 nach oben offen war, ist die bei der Anordnung 103 verwendete Umgebung 109, d.h. der jeweils verwendete Elektrolyt, in die Kammer 105 eingedrungen.

Dies hat zur Folge, daß die Zelle 107 auf der Anordnung 103 nur über eine Kontaktfläche aufliegt, die der ringförmigen Oberfläche der Beschichtung 106 im Bereich der Öffnung 108 entspricht. Die Elektrodenanordnung 103 steht demgegenüber mit dem Elektrolyten 109 über die gesamte Oberfläche der Beschichtung 106 in Verbindung, so daß diese Oberfläche wesentlich größer ist.

Es versteht sich dabei, daß die Anordnung gemäß Fig. 7 ebenfalls nur beispielhaft zu verstehen ist. Statt der dort dargestellten Anordnung kann ebenso gut ein Schwamm aus einem Edelmetall verwendet werden, bspw. ein Platinschwamm.

### Beispiel 1:

In einer Platte wurden 96 Mikroküvetten mit schrägen Wänden angebracht. Im Boden der Mikroküvetten wurden Elektroden eingegossen. Die Elektroden bestanden aus Golddraht, aufgerauht durch Ätzen, mit 20 µm Durchmesser und 10 µm Überstand. Der Überstand auf der Unterseite betrug 200 µm. Eine Signalverarbeitungs-Platte unterhalb der mit den Küvetten versehenen Deckplatte war mit SMD-Impedanzwandlern sowie Verstärkern versehen. Referenzelektroden mit einer Impedanz von 1 kΩ waren alle auf einen Punkt kontaktiert. Es wurden Nervenzellen aus embryonalem Hühnchen-Gehirn enzymatisch dissoziiert und in die Küvetten pipettiert. Die Zellen sanken auf die Elektroden ab und bildeten dort Aggregate mit vernetzten Zellstrukturen. Die Signalamplitude lag bei 200 µV.

### Beispiel 2:

Eine Deckplatte wurde mit 192 Mikroküvetten mit konischen Wänden versehen. Die Bodenöffnung betrug 100 µm im Durchmesser. Die Wände der Küvetten waren silikonisiert. Am Boden der Küvetten befanden sich Elektrodenplatten mit Flächenelektroden von 1 mm Durchmesser. Diese waren auf einer Keramikplatte dickschichttechnisch hergestellt. Die Oberfläche der Elektroden war galvanisch platiniert. Durch Verbindung der Deckplatte mit der Elektroden-Platte wurden die effektiven Elektrodenflächen auf 100 µm reduziert, d.h. von 10 kΩ auf 1 MΩ. Nach dem Einbringen von Hühnchenzellen, der Bildung von Aggregaten und dem Absinken der Zellen auf die Elektroden (vollständige Bedeckung) wurde ein ausreichendes Signal/Rausch-Verhältnis bis zu einer Signalspannung von 4 mV erzielt.

### Beispiel 3:

In einer Deckplatte wurden 200 Mikroküvetten mit Öffnungen am Boden von jeweils 50 µm Durchmesser ausgebildet. Eine Elektroden-Platte wurde mit Leiterbahnen von 10 µm Breite im Abstand von 50 µm versehen. Die Leiterbahnen waren nicht isoliert. Sie wurden hergestellt, indem Gold galvanisch aus einer Goldchlorid-Lösung bis zu einer Elektrodenimpedanz von 100 kΩ abgeschieden wurde. Die Orientierung der Leiterbahnen verlief senkrecht zu den Achsen der Mikroküvetten. Die Deckplatte wurde auf die Elektroden-Platte geklemmt. Eine Zwischengummierung war zum Abdichten vorgesehen. Es entstanden 3 bis 4 Elektroden von 10 x 50 µm, die die Ableitsicherheit erhöhten. Die Messungen wurden jeweils differenziell gegen eine für jede Kammer von oben eingeführte Referenzelektrode durchgeführt. Es wurden Neuroblastoma-Zellen aus Anzuchtkulturen passagiert. Zusätzlich wurde ein elektrisches Feld angelegt, um eine wanderungsbewegung der Zellen auf die Elektroden zu zu bewirken.

### Beispiel 4:

Die Mikroküvetten waren mit einem Loch von 0.5 mm Durchmesser versehen. Die schrägen Wände der Mikroküvetten waren silikonisiert. Zwischen den Platten wurde eine Gummierung zur Abdichtung eingesetzt. Eine Elektroden-Platte wurde zur Ausbildung von Flächenelektroden mit 2 mm Durchmesser galvanisch mit Goldmohr überzogen (10 kΩ).

Insgesamt erscheinen Anordnungen mit 2 bis zu mehreren 1 . 000 Mikroküvetten möglich. Die Mikroküvetten haben dabei ein Volumen zwischen 1 µl und 100 ml. Die Elektrodenfläche kann einen Durchmesser zwischen 1 µm und 1 mm haben.

Insgesamt wird durch die Erfindung ermöglicht, einzelne Zellen oder Zellaggregate aktiv an bestimmte Zellen eines Multiableitelektrodenarrays und/oder eines Multiküvettenarrays zu positionieren. Potentielle Anwendungen der Erfindung liegen im Bereich der Pharmakologie, des Pharmascreenings, der Neurobiologie und der Biosensorik.

## Patentansprüche

1. Mikroelementennanordnung mit einer Vielzahl von auf einem Substrat (15, 16; 40; 51, 52; 90, 91; 95, 96, 97; 104) angeordneten elektrischen Mikroelementen zum Kontaktieren von in einer flüssigen Umgebung (30; 70; 109) befindlichen Zellen (31; 42; 71), **gekennzeichnet durch** Mittel zum Führen und/oder Vereinzeln und/oder mechanischen Anziehen der Zellen (31; 71) an die Mikroelemente (21; 61; 85, 86, 87).

2. Anordnung nach Anspruch 1, **dadurch gekennzeichnet, daß** die Mikroelemente (21; 41; 61; 85, 86, 87; 103) zum Ableiten von bioelektrischen Potentialen und/oder zum bioelektrischen Stimulieren der Zellen (31; 42; 71; 107) vorgesehen sind.

3. Anordnung nach Anspruch 1, **dadurch gekennzeichnet, daß** die Mikroelemente als lichtempfindliche Elemente ausgebildet sind.

4. Anordnung nach einem oder mehreren der Ansprüche 1 bis 3, **dadurch gekennzeichnet, daß** die Mittel eine Unterdruck-Kraft auf die Zellen (31) ausüben.

5. Anordnung nach einem oder mehreren der Ansprüche 1 bis 3, **dadurch gekennzeichnet, daß** die Mittel eine hydrodynamische Kraft auf die Zellen (71) ausüben.

6. Anordnung nach Anspruch 4 oder 5, **dadurch gekennzeichnet, daß** die Mittel Kanäle (22; 62) umfassen, die in einer Kontaktoberfläche (24; 64) der Mikroelektroden (21; 61) ausmünden.

7. Anordnung nach Anspruch 4 und 6, **dadurch gekennzeichnet, daß** die Kanäle (22) mit einer Unterdruckquelle verbindbar sind.

8. Anordnung nach Anspruch 5 und 6, **dadurch gekennzeichnet, daß** die Kanäle (62) mit einer Pumpeinrichtung (65, 66, 67) für die flüssige biologische Umgebung (70) verbunden sind.

9. Anordnung nach Anspruch 8, **dadurch gekennzeichnet, daß** die Pumpeinrichtung als Elektroosmose-Einrichtung ausgebildet ist.

10. Anordnung nach Anspruch 9, **dadurch gekennzeichnet, daß** die Pumpeinrichtung (65, 66, 67) als Elektroosmose-Einrichtung zwei Elektroden (66, 67) umfaßt, die an entgegengesetzten Enden der Kanäle (62) wirksam sind, und daß zwischen die Elektroden (66, 67) eine Spannung geschaltet ist.

11. Anordnung nach Anspruch 10, **dadurch gekennzeichnet, daß** die Mittel eine elektrostatische Kraft (E) auf die Zellen ausüben.

12. Anordnung, nach einem oder mehreren der Ansprüche 1 bis 11, **gekennzeichnet durch** Mittel zum Führen und/oder Vereinzeln der Zellen (31; 71) vor dem mechanischen Anziehen an die Mikroelemente.

13. Anordnung nach einem oder mehreren der Ansprüche 1 bis 12, **dadurch gekennzeichnet, daß** die Mittel trichterartige Mikroküvetten (20; 60; 84) im Substrat (15, 16; 51, 52; 90, 91; 95, 96, 97) umfassen, wobei sich die Mikroelemente am Boden der Mikroküvetten (20; 60; 84) befinden.

14. Anordnung nach Anspruch 13, **dadurch gekennzeichnet, daß** Oberflächenbereiche zwischen den Mikroküvetten mit einem zellabweisenden Substrat und/oder die Mikroküvetten mit einem zellanziehenden Substrat beschichtet sind.

15. Anordnung nach einem oder mehreren der Ansprüche 1 bis 14, **dadurch gekennzeichnet, daß** das Substrat (15, 16; 51, 52; 90, 91; 95, 96, 97) mindestens aus einer Grundplatte (15; 51; 90; 95, 96) und einer darüberliegenden Deckplatte (16; 52; 91; 97) besteht.

16. Anordnung nach Anspruch 15, **dadurch gekennzeichnet, daß** die Grundplatte (15; 51; 90; 95, 96) aus Glas, Quarz, Silizium oder Kunststoff besteht.

17. Anordnung nach Anspruch 15 oder 16, **dadurch gekennzeichnet, daß** die Deckplatte (16; 52; 91; 97) aus Glas, Quarz, Silizium oder Kunststoff, insbesondere aus Polystyrol, PMMA oder Polyimid besteht.

18. Anordnung nach einem oder mehreren der Ansprüche 15 bis 17, **dadurch gekennzeichnet, daß** die Grundplatte (15; 51; 90; 95, 96) und/oder die Deckplatte (16; 52; 91; 97) aus einem für Licht durchlässigen Material besteht, wobei die Wellenlänge des Lichtes in einem für Mikroskopietechniken zugänglichen Bereich des Spektrums liegt.

19. Anordnung nach einem oder mehreren der Ansprüche 15 bis 18, **dadurch gekennzeichnet, daß** die mit den Mikroelementen versehene Grundplatte (90) seitlich als Steckerleiste (82) herausgeführt ist.

20. Anordnung nach einem oder mehreren der Ansprüche 15 bis 18, **dadurch gekennzeichnet, daß** die Grundplatte (95) mindestens einer unteren Signalverarbeitungs-Platte (95) und einer darüberliegenden Elementen-Platte (96) besteht.

21. Anordnung nach Anspruch 20, **dadurch gekennzeichnet, daß** die Signalverarbeitungs-Platte (95) seitlich als Steckerleiste (82) herausgeführt ist.

22. Anordnung nach einem oder mehreren der Ansprüche 2 bis 21, **dadurch gekennzeichnet, daß** die Mikroelektroden (85, 86, 87) Ableitelektroden (85) sowie Reizelektroden (86) und/oder Referenzelektroden (87) umfassen.

23. Anordnung nach Anspruch 22, **dadurch gekennzeichnet, daß** die Mikroelektroden (85, 86, 87) mehrere konzentrisch angeordnete Einzelelektroden umfassen.

24. Anordnung nach Anspruch 13 und 22, **dadurch gekennzeichnet, daß** die Referenzelektrode (87a) im Abstand oberhalb der am Boden der Mikroküvette (84a) angeordneten Ableitelektrode (85a) angeordnet ist.

25. Anordnung nach Anspruch 2, **dadurch gekennzeichnet, daß** die mit der Umgebung (109) in Kontakt befindliche Oberfläche der Mikroelektrode (103) größer ist als die mit der Zelle (107) in Kontakt befindliche Oberfläche.

26. Anordnung nach Anspruch 25, **dadurch gekennzeichnet, daß** die Mikroelektrode (103) als Kammer (105) in einem Substrat (104) ausgebildet ist, wobei die Kammer (105) über eine Öffnung (108) in den das Substrat (104) umgebenden Außenraum mündet.

27. Verfahren zum Kontaktieren von in einer flüssigen Umgebung oberhalb eines Substrates (15, 16; 40; 51, 52; 90, 91; 95, 96, 97) befindlichen Zellen (31; 42; 71), bei dem ein Kontakt zwischen den Zellen (31; 42; 71) und elektrischen Mikroelementen hergestellt wird, **dadurch gekennzeichnet, daß** eine Führungs- und/oder Anziehungskraft zwischen den Zellen (31; 71) und den Mikroelementen oder dem Substrat (15, 16; 51, 52; 90, 91; 95, 96, 97) erzeugt wird.

28. Verfahren nach Anspruch 27, **dadurch gekennzeichnet, daß** die Kraft als Unterdruck-Kraft ausgeübt wird.

29. Verfahren nach Anspruch 28, **dadurch gekennzeichnet, daß** die Kraft als hydrodynamische Kraft ausgeübt wird.

30. Verfahren nach Anspruch 29, **dadurch gekennzeichnet, daß** die hydrodynamische Kraft mittels Elektroosmose, insbesondere durch einen mittels Elektroosmose erzeugten Elektrolytfluß, ausgeübt wird.

31. Verfahren nach Anspruch 27, **dadurch gekennzeichnet, daß** die Kraft als Anziehungskraft aufgrund elektrischer Ladung der Zellen und eines in Richtung der Elektroden (85a) wirkenden elektrischen Feldes (E) ausgeübt wird.

32. Verfahren nach einem oder mehreren der Ansprüche 27 bis 31, **dadurch gekennzeichnet, daß** die Anziehungskraft als Kontaktkraft, insbesondere durch Ansaugen, zwischen Zellen (31; 71) und Mikroelementen ausgeübt wird.

33. Verfahren nach einem oder mehreren der Ansprüche 27 bis 32, **dadurch gekennzeichnet, daß** die Kraft für eine gerichtete Bewegung der Zellen (31; 71) auf die Mikroelemente zu ausgeübt wird.

34. Verfahren nach einem oder mehreren der Ansprüche 27 bis 33, **dadurch gekennzeichnet, daß** die Zellen (31; 71) über die als Mikroelektroden (21; 61; 86) ausgebildeten Mikroelemente stimuliert werden.

35. Verfahren nach einem oder mehreren der Ansprüche 27 bis 33, **dadurch gekennzeichnet, daß** über die als Mikroelektroden (21; 61; 85) ausgebildeten Mikroelemente Potentiale von den Zellen (31; 71) abgeleitet werden.

36. Verfahren nach einem oder mehreren der Ansprüche 27 bis 33, **dadurch gekennzeichnet, daß** über die als Mikrophotodioden ausgebildeten Mikroelemente die Lumineszenz der Zellen und/oder deren Lichtabsorption gemessen wird.

37. Verfahren zum Herstellen einer Mikroelementenanordnung mit einer Vielzahl von elektrischen Mikroelementen gemäß Ansprüchen 1 bis 26, bei dem die Mikroelemente auf einem Substrat (15, 16; 40; 51, 52; 90, 91; 95, 96, 97) angeordnet werden, **dadurch gekennzeichnet, daß** das Substrat (15, 16; 51, 52; 90, 91; 95, 96, 97) mindestens aus einer Grundplatte (15; 51; 90; 95, 96) und einer darüberliegenden Deckplatte (16; 52; 91; 97) hergestellt wird.

38. Verfahren nach Anspruch 37, **dadurch gekennzeichnet, daß** in der Grundplatte (15; 51) ein Kanalsystem (23; 62, 63) ausgebildet wird, daß in der Deckplatte (16; 52) Mikroküvetten (20; 60) ausgeformt werden, und daß die Grundplatte (15; 51) mit der Deckplatte (16; 52) derart zusammengefügt wird, daß Öffnung (22) am Boden der Mikroküvetten (20; 60) in Kontaktoberflächen (24; 64) der Mikroelemente angeordnet sind und mit dem Kanalsystem (23; 62, 63) kommunizieren.

39. Verfahren nach Anspruch 36 oder 37, **gekennzeichnet durch** die Schritte:
a) Versehen von Grundplatte (15; 51; 90; 95, 96) und/oder Deckplatte (16; 52; 91; 97) auf ihren einander zu weisenden Oberflächen mit einer Schicht von Molekülen mit reaktiver Endgruppe;
b) Zusammenfügen von Grundplatte (15; 51; 90; 95, 96) und Deckplatte (16; 52; 91; 97); und
c) Aktivieren einer kovalenten Bindung der Schichten mittels eines äußeren Stimulus.

40. Verfahren nach Anspruch 39, **dadurch gekennzeichnet, daß** nach Schritt b) die Grundplatte (15; 51; 90; 95, 96) und die Deckplatte (16; 52; 91; 97) relativ zueinander justiert werden.

41. Verfahren nach Anspruch 39 oder 40, **dadurch gekennzeichnet, daß** der äußere Stimulus als Temperatur, Licht oder elektrisches Feld ausgeübt wird.

42. Verfahren nach Anspruch 38, **dadurch gekennzeichnet, daß** die Grundplatte (15; 51; 90; 95, 96) und die Deckplatte (16; 52; 91; 97) durch anodisches oder metallisches Bonden miteinander verbunden werden.

## Claims

1. A microelement arrangement having a plurality of electrical microelements, arranged on a substrate (15, 16; 40; 51, 52; 90, 91; 95, 96, 97; 104), for making contact to cells (31; 42; 71) present in a liquid environment (30; 70; 109), **characterized by** means for guiding and/or isolating and/or mechanically attracting the cells (31; 71) onto the microelements (21; 61; 85, 86, 87).

2. The arrangement of claim 1, **characterized in that** the microelements (21; 41; 61; 85, 86, 87; 103) are provided for sensing bioelectric potentials and/or for bioelectrical stimulation of said cells (31; 42; 71; 107).

3. The arrangement of claim 1, **characterized in that** the microelements are configured as light-sensitive elements.

4. The arrangement of one or more of claims 1 through 3, **characterized in that** the means exert a negative-pressure force on the cells (31).

5. The arrangement of one or more of claims 1 through 3, **characterized in that** the means exert a hydrodynamic force on the cells (71).

6. The arrangement of claim 4 or claim 5, **characterized in that** the means comprise channels (22; 62) which open out at a contact surface (24; 64) of the microelectrodes (21; 61).

7. The arrangement of claims 4 and 6, **characterized in that** the channels (22) can be connected to a source of negative-pressure.

8. The arrangement of claim 5 and claim 6, **characterized in that** the channels (62) are connected to a pump device (65, 66, 67) for the liquid biological environment (70).

9. The arrangement of claim 8, **characterized in that** the pump device is configured as an electroosmosis device.

10. The arrangement of claim 9, **characterized in that** the pump device (65, 66, 67) comprises as said electroosmosis device two electrodes (66, 67) which are effective at opposite ends of the channels (62), and that a voltage is applied between the electrodes (66, 67).

11. The arrangement of claim 10, **characterized in that** the means exert an electrostatic force (E) on the cells.

12. The arrangement of one or more of claims 1 through 11, **characterized by** means for guiding and/or isolating said cells (31; 71) prior to mechanical attraction onto the microelements.

13. The arrangement of one or more of claims 1 through 12, **characterized in that** the means comprise funnel-like microcuvettes (20; 60; 84) within said substrate (15, 16; 51, 52; 90, 91; 95, 96, 97), whereby the microelements are located at the bottom of the microcuvettes (20; 60; 84).

14. The arrangement of claim 13, **characterized in that** surface regions between said microcuvettes are coated with a cell-repelling substrate and/or that the microcuvettes are coated with a cell-attracting substrate.

15. The arrangement of one or more of claims 1 through 14, **characterized in that** said substrate (15, 16; 51, 52; 90, 91; 95, 96, 97) comprises at least a base plate (15; 51; 90; 95, 96) and a cover plate (16; 52; 91; 97) located thereabove.

16. The arrangement of claim 15, **characterized in that** the base plate (15; 51; 90; 95, 96) is made of glass, quartz, silicon, or plastic.

17. The arrangement of claim 15 or claim 16, **characterized in that** the cover plate (16; 52; 91; 97) is made of glass, quartz, silicon, or plastic, in particular of polystyrene, PMMA, or polyimide.

18. The arrangement of one or more of claims 15 through 17, **characterized in that** the base plate (15; 51; 90; 95, 96) and/or the cover plate (16; 52; 91; 97) are made of a material that is transparent to light, whereby the wavelength of the light is in a region of the spectrum accessible to microscopy techniques.

19. The arrangement of one or more of claims 15 through 18, **characterized in that** the base plate (90) equipped with the microelements is guided outward laterally as an edge connector (82).

20. The arrangement of one or more of claims 15 through 18, **characterized in that** the base plate (95) comprises at least a lower signal processing plate (95) and an element plate (96) located thereabove.

21. The arrangement of claim 20, **characterized in that** the signal processing plate (95) projects outward laterally as an edge connector (82).

22. The arrangement of one or more of claims 2 through 21, **characterized in that** the microelectrodes (85, 86, 87) comprise sensing electrodes (85) as well as stimulus electrodes (86) and/or reference electrodes (87).

23. The arrangement of claim 22, **characterized in that** the microelectrodes (85, 86, 87) comprise multiple concentrically arranged individual electrodes.

24. The arrangement of claim 13 and claim 22, **characterized in that** the reference electrode (87a) is arranged at a distance above the sensing electrode (85a) which is arranged at the bottom of the microcuvette (84a).

25. The arrangement of claim 2, **characterized in that** the surface of the microelectrode (103) in contact with the environment (109) is greater than the surface which is in contact with said cell (107).

26. The arrangement of claim 25, **characterized in that** the microelectrode (103) is configured as a chamber (105) within said substrate (104), whereby said chamber (105) communicates via an opening (108) into the external space surrounding the substrate (104).

27. A method for making contact to cells (31; 42; 71) present in a liquid environment above a substrate (15, 16; 40; 51, 52; 90, 91; 95, 96, 97), whereby a contact is created between said cells (31; 42; 71) and electrical microelements, **characterized in that** a guiding force and/or attractive force is generated between said cells (31; 71) and the microelements or the substrate (15, 16; 51, 52; 90, 91; 95, 96, 97).

28. The method of claim 27, **characterized in that** the force is exerted as a negative-pressure force.

29. The method of claim 28, **characterized in that** said force is exerted as hydrodynamic force.

30. The method of claim 29, **characterized in that** the hydrodynamic force is exerted by electroosmosis, in particular by way of an electrolyte flow generated by electroosmosis.

31. The method of claim 27, **characterized in that** said force is exerted as an attractive force on the basis of an electrical charge of said cells and an electrical field (E) acting in the direction of the electrodes (85a).

32. The method of one or more of claims 27 through 31, **characterized in that** the attractive force is exerted as a contact force, in particular by aspiration, between cells (31; 71) and microelements.

33. The method of one or more of claims 27 through 32, **characterized in that** said force is exerted for a directed movement of said cells (31; 71) towards the microelements.

34. The method of one or more of claims 27 through 33, **characterized in that** said cells (31; 71) are stimulated via said microelements that are configured as microelectrodes (21; 61; 86).

35. The method of one or more of claims 27 through 33, **characterized in that** via said microelements that are configured as microelectrodes (21; 61; 85) potentials of said cells (31; 71) are sensed.

36. The method of one or more of claims 27 through 33, **characterized in that** via said microelements configured as microphotodiodes the luminescence of said cells and/or their light absorption are measured.

37. A method for manufacturing a microelement arrangement having a plurality of electrical microelements according to claims 1 - 26, wherein said microelements are arranged on a substrate (15, 16; 40; 51, 52; 90, 91; 95, 96, 97), **characterized in that** said substrate (15, 16; 51, 52; 90, 91; 95, 96, 97) is manufactured at least from a base plate (15; 51; 90; 95, 96) and a cover plate (16; 52; 91; 97) located thereabove.

38. The method of claim 37, **characterized in that** a channel system (23; 62, 63) is configured within said base plate (15; 61), that microcuvettes (20; 60) are shaped within said cover plate (16; 52), and that said base plate (15; 51) is fitted to the cover plate (16; 52) such that openings (22) at the bottom of the microcuvettes (20; 60) are arranged within contact surfaces (24; 64) of said microelements and communicate with the channel system (23; 62, 63).

39. The method of claim 36 or claim 37, **characterized by** the steps:
a) providing said base plate (15; 51; 90; 95, 96) and/or said cover plate (16; 52; 91; 97) on their surfaces facing one another with a layer of molecules having a reactive terminal group;
b) fitting said base plate (15; 51; 90; 95, 96) and said cover plate (16; 52; 91; 97) together; and
c) activating a covalent bond between said layers by way of an external stimulus.

40. The method of claim 39, **characterized in that** after step b) the base plate (15; 51; 90; 95, 96) and the cover plate (16; 52; 91; 97) are adjusted relative to one another.

41. The method of claim 39 or claim 40, **characterized in that** said external stimulus is exerted as temperature, light or electric field.

42. The method of claim 38, **characterized in that** said base plate (15; 51; 90; 95, 96) and said cover plate (16; 52; 91; 97) are joined to one another by anodic or metallic bonding.

## Revendications

1. Système de micro-éléments comportant une pluralité de micro-éléments déposés sur un substrat (15, 16 ; 40 ; 51, 52 ; 90, 91 ; 95, 96, 97 ; 104) destinés à être mis en contact avec des cellules (31 ; 42 ; 71) situées dans un environnement liquide (30 ; 70 ; 109), **caractérisé par** des moyens de guidage et/ou de séparation et/ou d'attraction mécanique des cellules (31 ; 71) vers les micro-éléments (21 ; 61 ; 85, 86, 87).

2. Système selon la revendication 1, **caractérisé en ce que** les micro-éléments (21 ; 41 ; 61 ; 85, 86, 87 ; 103) sont prévus pour la dérivation des potentiels bioélectriques et/ou pour la stimulation bioélectrique des cellules (31 ; 42 ; 71 ; 107).

3. Système selon la revendication 1, **caractérisé en ce que** les micro-éléments sont réalisés sous forme d'éléments photosensibles.

4. Système selon une ou plusieurs des revendications 1 à 3, **caractérisé en ce que** les moyens exercent une force de dépression sur les cellules (31).

5. Système selon une ou plusieurs des revendications 1 à 3, **caractérisé en ce que** les moyens exercent une force hydrodynamique sur les cellules (71).

6. Système selon la revendication 4 ou 5, **caractérisé en ce que** les moyens comportent des canaux (22 ; 62) qui débouchent dans une surface de contact (24 ; 64) des micro-électrodes (21 ; 61).

7. Système selon les revendications 4 et 6, **caractérisé en ce que** les canaux (22) peuvent être raccordés à une source de dépression.

8. Système selon les revendications 5 et 6, **caractérisé en ce que** les canaux (62) sont raccordés à une installation de pompe (65, 66, 67) pour l'environnement biologique liquide (70).

9. Système selon la revendication 8, **caractérisé en ce que** l'installation de pompe est conçue sous forme d'installation d'électroosmose.

10. Système selon la revendication 9, **caractérisé en ce que** l'installation de pompe (65, 66, 67) comporte deux électrodes (66, 67) formant l'installation d'électro-osmose, qui sont actives sur des extrémités opposées des canaux (62), et **en ce qu'**une tension est appliquée entre les électrodes (66, 67).

11. Système selon la revendication 10, **caractérisé en ce que** les moyens exercent une force électrostatique (E) sur les cellules.

12. Système selon une ou plusieurs des revendications 1 à 11,
**caractérisé par** des moyens de guidage et/ou de séparation des cellules (31 ; 71) avant l'attraction mécanique vers les micro-éléments.

13. Système selon une ou plusieurs des revendications 1 à 12, **caractérisé en ce que** les moyens comportent des micro-cuvettes (20 ; 60 ; 84) en forme de trémie dans le substrat (15, 16 ; 51, 52 ; 90, 91 ; 95, 96, 97), les micro-éléments étant situés au fond des micro-cuvettes (20 ; 60 ; 84).

14. Système selon la revendication 13, **caractérisé en ce que** des zones de surface entre les micro-cuvettes sont revêtues d'un substrat repoussant les cellules et/ou les micro-cuvettes sont revêtues d'un substrat attirant les cellules.

15. Système selon une ou plusieurs des revendications 1 à 14, **caractérisé en ce que** le substrat (15, 16 ; 51, 52 ; 90, 91 ; 95, 96, 97) est formé au moins par une plaque de base (15 ; 51 ; 90 ; 95, 96) et une plaque de recouvrement (16 ; 52 ; 91 ; 97) disposée sur celle-ci.

16. Système selon la revendication 15, **caractérisé en ce que** la plaque de base (15 ; 51 ; 90 ; 95, 96) est réalisée en verre, quartz, silicium ou matière synthétique.

17. Système selon la revendication 15 ou 16, **caractérisé en ce que** la plaque de recouvrement (16 ; 52 ; 91 ; 97) est réalisée en verre, quartz, silicium ou matière synthétique, en particulier en polystyrène, PMMA ou polyimide.

18. Système selon une ou plusieurs des revendications 15 à 17, **caractérisé en ce que** la plaque de base (15 ; 51 ; 90 ; 95, 96) et/ou la plaque de recouvrement (16 ; 52 ; 91 ; 97) sont réalisées dans un matériau laissant passer la lumière, la longueur d'onde de la lumière étant située dans une zone du spectre accessible aux techniques de microscopie.

19. Système selon une ou plusieurs des revendications 15 à 18, **caractérisé en ce que** la plaque de base (90), munie des micro-éléments, est guidée latéralement vers l'extérieur sous forme de rebord de connexion (82).

20. Système selon une ou plusieurs des revendications 15 à 18, **caractérisé en ce que** la plaque de base (95) est formée au moins par une plaque de traitement des signaux (95) inférieure et une plaque d'éléments (96) disposée sur celle-ci.

21. Système selon la revendication 20, **caractérisé en ce que** la plaque de traitement des signaux (95) est guidée latéralement vers l'extérieur sous forme de rebord de connexion (82).

22. Système selon une ou plusieurs des revendications 2 à 21, **caractérisé en ce que** les micro-électrodes (85, 86, 87) comportent des électrodes de dérivation (85), ainsi que des électrodes de stimulation (86) et/ou des électrodes de référence (87).

23. Système selon la revendication 22, **caractérisé en ce que** les micro-électrodes (85, 86, 87) comportent plusieurs électrodes individuelles disposées concentriquement.

24. Système selon les revendications 13 et 22, **caractérisé en ce que** l'électrode de référence (87a) est disposée à distance au-dessus de l'électrode de dérivation (85a) disposée sur le fond de la microcuvette (84a).

25. Système selon la revendication 2, **caractérisé en ce que** la surface de la micro-électrode (103), se trouvant en contact avec l'environnement (109), est supérieure à la surface se trouvant en contact avec la cellule (107).

26. Système selon la revendication 25, **caractérisé en ce que** la micro-électrode (103) est réalisée sous forme de chambre (105) dans un substrat (104), la chambre (105) débouchant par l'intermédiaire d'une ouverture (108) dans l'espace extérieur entourant le substrat (104).

27. Procédé de mise en contact de cellules (31 ; 42 ; 71) situées dans un environnement liquide au-dessus d'un substrat (15, 16 ; 40 ; 51, 52 ; 90, 91 ; 95, 96, 97), dans lequel un contact est établi entre les cellules (31 ; 42 ; 71) et des micro-éléments électriques, **caractérisé en ce qu'**une force de guidage et/ou d'attraction est générée entre les cellules (31 ; 71) et les micro-éléments ou le substrat (15, 16 ; 51, 52 ; 90, 91 ; 95, 96, 97).

28. Procédé selon la revendication 27, **caractérisé en ce que** la force exercée est une force de dépression.

29. Procédé selon la revendication 28, **caractérisé en ce que** la force exercée est une force hydrodynamique.

30. Procédé selon la revendication 29, **caractérisé en ce que** la force hydrodynamique est exercée au moyen d'une électro-osmose, en particulier par un flux d'électrolyte généré par électro-osmose.

31. Procédé selon la revendication 27, **caractérisé en ce que** la force exercée est une force d'attraction en raison d'une charge électrique des cellules et d'un champ électrique (E) agissant en direction des électrodes (85a).

32. Procédé selon une ou plusieurs des revendications 27 à 31, **caractérisé en ce que** la force d'attraction exercée est une force de contact, en particulier par aspiration, entre les cellules (31 ; 71) et des micro-éléments.

33. Procédé selon une ou plusieurs des revendications 27 à 32, **caractérisé en ce que** la force est exercée pour un mouvement des cellules (31 ; 71) dirigé sur les micro-éléments.

34. Procédé selon une ou plusieurs des revendications 27 à 33, **caractérisé en ce que** les cellules (31 ; 71) sont stimulées par des micro-éléments réalisés sous forme de micro-électrodes (21 ; 61 ; 86).

35. Procédé selon une ou plusieurs des revendications 27 à 33, **caractérisé en ce que** des potentiels sont dérivés des cellules (31 ; 71) par l'intermédiaire des micro-éléments concus sous forme de micro-électrodes (21 ; 61 ; 85).

36. Procédé selon une ou plusieurs des revendications 27 à 33, **caractérisé en ce que** la luminescence des cellules et/ou leur absorption de lumière est mesurée par l'intermédiaire des micro-éléments réalisés sous forme de micro-photodiodes.

37. Procédé de réalisation d'un système de micro-éléments comportant une pluralité de micro-éléments électriques selon les revendications 1 à 26, dans lequel les micro-éléments sont déposés sur un substrat (15, 16 ; 40 ; 51, 52 ; 90, 91 ; 95, 96, 97), **caractérisé en ce que** le substrat (15, 16 ; 51, 52 ; 90, 91 ; 95, 96, 97) est réalisé au moins avec une plaque de base (15 ; 51 ; 90 ; 95, 96) et une plaque de recouvrement (16 ; 52 ; 91 ; 97) disposée sur celle-ci.

38. Procédé selon la revendication 37, **caractérisé en ce qu'**un système de canaux (23 ; 62, 63) est réalisé dans la plaque de base (15 ; 51), **en ce que** des micro-cuvettes (20 ; 60) sont formées dans la plaque de recouvrement (16; 52), et **en ce que** la plaque de base (15; 51) est assemblée avec la plaque de recouvrement (16 ; 52) de telle sorte que des ouvertures (22) sont ménagées dans le fond des micro-cuvettes (20 ; 60) dans des zones de contact (24 ; 64) des micro-éléments et communiquent avec le système de canaux (23 ; 62, 63).

39. Procédé selon la revendication 36 ou 37, **caractérisé par** les étapes :
a) application d'une couche de molécules avec des groupes terminaux réactifs sur la plaque de base (15 ; 51 ; 90 ; 95, 96) et/ou la plaque de recouvrement (16 ; 52 ; 91 ; 97), sur leurs surfaces orientées l'une vers l'autre ;
b) assemblage de la plaque de base (15 ; 51 ; 90 ; 95, 96) et de la plaque de recouvrement (16 ; 52 ; 91 ; 97) ; et
c) activation d'une liaison covalente des couches au moyen d'un stimulus externe.

40. Procédé selon la revendication 39, **caractérisé en ce que**, après l'étape b), la plaque de base (15 ; 51 ; 90 ; 95, 96) et la plaque de recouvrement (16 ; 52 ; 91 ; 97) sont ajustées l'une par rapport à l'autre.

41. Procédé selon la revendication 39 ou 40, **caractérisé en ce que** le stimulus externe est exercé sous forme de température, de lumière ou de champ électrique.

42. Procédé selon la revendication 38, **caractérisé en ce que** la plaque de base (15 ; 51 ; 90 ; 95, 96) et la plaque de recouvrement (16 ; 52 ; 91 ; 97) sont assemblées entre elles par une liaison anodique ou métallique.
